# EUROPEAN PATENT APPLICATION

(11) **EP 4 663 138 A1**
(43) Date of publication of application: **17.12.2025**
(21) Application number: 24182425.9
(22) Date of filing: 14.06.2024
(51) Int. Cl.: A61B 17/3201

(54) **SURGICAL INSTRUMENT**

(71) Applicant: SNE Swiss Neuro Engineering AG, 8260 Stein am Rhein (CH)
(72) Inventor: SCHALLER, Philipp, 8260 Stein am Rhein (CH); VEZZÙ, Guido, 8422 Pfungen (CH)
(74) Representative: Schulz Junghans Patentanwälte PartGmbB

(57) **Abstract**

The present invention relates to a surgical instrument (1), comprising a tube (2) and a handle (7) operatively connected thereto, wherein for facilitating an unobstructed view along a top side (22a) of the tube (2) in the direction of a longitudinal axis (x) of the tube, the surgical instrument (1) comprises at least one operating state in which the surgical instrument (1) extends no more than 4 mm beyond a maximum apex (22c) of the top side (22a) of the tube (2) in the direction of the vertical axis (z) in any cross-section of the surgical instrument (1) that extends perpendicular to the longitudinal axis (x) and is arranged between the distal end (2b) of the tube (2) and an opposing end (1a) of the surgical instrument (1).

## Description

The present invention relates to a surgical instrument.

In surgical fields such as neurosurgery as well as other delicate surgical procedures, particularly under the microscope, the visibility is usually limited due to the small space conditions and the required orientation of the instruments.

Particularly, neurosurgery involves the detection and surgical treatment of diseases, injuries and malformations of the central nervous system and its sheaths. This primarily includes the head (cranial) and the spinal column (spinal).

In neurosurgery, the procedures are macroscopic in nature, but are supplemented by microsurgical techniques.

To ensure minimally invasive but maximally effective neurosurgery while protecting important brain and nerve functions, the surgeon depends on having the right tools and instruments (of the necessary quality) available.

Particularly, the optimal tissue-instrument interaction and therefore the least atraumatic anatomy modification depends on optimal accessibility and visibility.

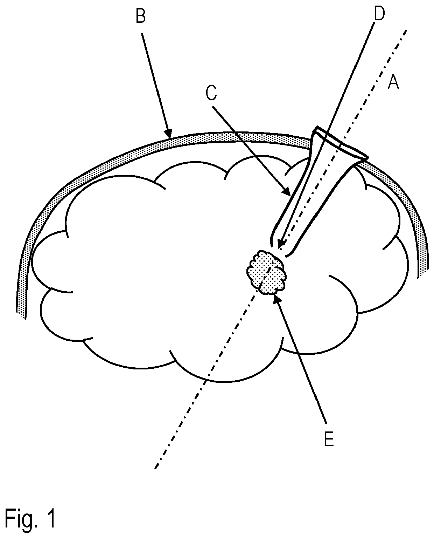

On the one hand, this is ensured using certain aids, such as brain access cannulas C as depicted in Fig. 1 which are inserted through an opening (e.g. in the skull B) and provide access to the site E of the procedure (e.g. for removal of a tumor).

Such access systems C, for example also widely known as trocar system, consist at least of a cannula and a guiding/protecting part inside during the insertion of the cannula. Once the access system C has been placed, the inner part is pulled out, opening the underside of the access system C. The surgeon then operates through this opening D.

The patient or the access system C is normally positioned so that the optical axis A of the microscope and the access system C coincide.

Due to the limited space available in these access systems, the design of the surgical instruments has a decisive influence on accessibility and visibility.

Based on the above, the problem to be solved by the present invention is to provide an improved visual access to the instrument-tissue interaction to the surgeon.

This problem is solved by a surgical instrument having the features of claim 1. Preferred embodiments are stated in the dependent claims and are described below.

According to claim 1, a surgical instrument is disclosed, comprising:
- a tube extending along a longitudinal axis from a proximal end to a distal end of the tube, wherein the tube comprises a circumferential wall delimiting a lumen of the tube, wherein the circumferential wall comprises an outer surface facing away from the lumen, the outer surface comprising a top side and a bottom side, and
- a handle for holding and manually operating the surgical instrument, the handle being operatively connected to the tube, the handle extending along a vertical axis of the surgical instrument on the bottom side of the tube, the vertical axis extending orthogonal to the longitudinal axis (and particularly crossing the longitudinal axis) and pointing away from the top side of the tube, wherein the bottom side is arranged below the top side of the tube in the direction of the vertical axis, and
wherein for allowing an unobstructed view along the top side of the tube in the direction of the longitudinal axis (e.g. from the proximal end to the distal end of the tube), the surgical instrument comprises at least one operating state in which the surgical instrument extends no more than 4mm, particularly no more than 3mm, particularly no more than 2mm, particularly no more than 1 mm, particularly no more than 0.5 mm beyond a maximum apex of the top side of the tube in the direction of the vertical axis in any cross-section of the surgical instrument that extends perpendicular to the longitudinal axis and is arranged between the distal end of the tube and an opposing end of the surgical instrument.

Particularly, the tube is an elongated tube delimiting an elongated lumen extending along the longitudinal axis.

Particularly, the notion of an operating state relates to the fully assembled surgical instrument and particularly relates to positions of one or several movable parts of the surgical instrument with respect to the tube. Thus, the surgical instrument comprises at least one operating state in which any such movable part (or any other part) does not extend past said top side in the manner described above.

Thus, the present invention seeks to reduce any distracting components above said top side of the tube of the surgical instrument. Due to this design, compared to conventional instruments, the surgical instrument according to the present invention requires much less space owing to the tubular design with minimal buildup, and therefore offers clear advantages in terms of freedom of movement, maneuverability and visibility in tight spaces, particularly in the afore-described access systems / cannulas.

In a preferred embodiment, for facilitating the unobstructed view along the top side of the tube in the direction of the longitudinal axis (e.g. from said proximal towards said distal end of the tube), the surgical instrument does not extend beyond the maximum apex of the top side of the tube in the direction of the vertical axis in any cross-section of the surgical instrument that extends perpendicular to the longitudinal axis and is arranged between the distal end of the tube and the opposing end of the surgical instrument.

Thus, particularly, the surgical instrument according to the present invention does have a minimal structure on the tube (or protruding past the tube) or no such structure at all, so that a direct view of the surgical area is guaranteed even at steep angles.

Furthermore, according to a preferred embodiment, the longitudinal axis is a cylinder axis of the tube (i.e. the tube comprises a cylindrical shape) and particularly extends through the center of each circular cross section of the tube perpendicular to the longitudinal axis. Thus, the outer surface of the circumferential wall is a cylinder mantle.

Furthermore, according to a preferred embodiment, the tube is formed in one piece, i.e., the tube is monolithic. Particularly, the tube can be formed from a homogenous material. Furthermore, in an embodiment, the tube is formed out of a metal, particularly a steel alloy.

According to an alternative embodiment, particularly in case the tube comprises an end section connected to an adjacent section of the tube via a shoulder (so that the end section comprises a smaller outer diameter than said adjacent section), the tube can be formed by connecting two tubes to one another (e.g. be welding or gluing), particularly tubes of different outer diameter. Alternatively, also in this case, the tube can be monolithic (see above).

In certain embodiments (see further down below), the tube may comprise a distal portion comprising the distal end of the tube that may be curved away from the longitudinal axis for further improving sight along the instrument (this may allow to also move a tool protruding past the distal end of the tube out of the way for clear sight along the tube). In such an embodiment, the tube can comprise an elongated proximal portion adjoining the distal portion and comprising the proximal end of the tube. This proximal portion of the tube can be cylindrical and extend along the longitudinal axis forming a cylinder axis with respect to the proximal portion of the tube. Also here, the circumferential wall portion corresponding to the proximal portion can comprise an outer surface that is a cylinder mantle.

Furthermore, according to preferred embodiment of the present invention, the above-described features according to which the surgical instrument does not extend past the maximum apex of the top side of the tube at all or by more than the stated amount, hold generally regardless of said at least one operating state of the tube. Thus, in certain preferred embodiments of the present invention, any part of the surgical instrument, particularly any movable part, will not extend past said apex of the top side in the manner described above at all or by more than the above-stated amount(s). Thus, in such an embodiment, said operating state does not need to be mentioned at all.

According to yet another preferred embodiment of the present invention, the tube is configured to guide a fluid medium and/or tissue of a patient (particularly a fluid medium carrying tissue of the patient). Thus, particularly, the surgical instrument is configured to draw off a liquid medium and/or discharge a liquid medium through an opening of the tube arranged at a distal end of the tube, wherein said opening can be located on the face side of the tube at the distal end of the tube or can be a lateral opening formed in said wall of the tube at the distal end of the tube. Furthermore, according to a preferred embodiment of the present invention, the surgical instrument comprises a movable blade configured to be moved back and forth in the lumen of the tube to shred material carried by the fluid medium sucked into the lumen through said opening.

According to yet another preferred embodiment of the present invention, the surgical instrument comprises a tool insert arranged at least partially in the lumen of the tube, so that the tube and the tool insert can move, particularly slide, relative to one another in the direction of the longitudinal axis to operate the tool insert.

Furthermore, in a preferred embodiment of the present invention, the surgical instrument comprises an actuating mechanism configured to move the tube and the tool insert relative to one another to operate the tool insert, wherein particularly the actuating mechanism is configured to move the tube back and forth in the direction of the longitudinal axis with respect to the tool insert to operate the tool insert, or to move the tool insert back and forth in the direction of the longitudinal axis with respect to the tube to operate the tool insert.

Further, in a preferred embodiment of the present invention, the surgical instrument comprises a slider, particularly extending along the vertical axis of the surgical instrument, wherein the slider is connected to the bottom side of the outer surface of the tube, wherein the actuating mechanism is configured to move the slider back and forth in the direction of the longitudinal axis with respect to the tool insert to operate the tool insert, wherein particularly the surgical instrument comprises a guiding member having an interior space, wherein particularly the tube is configured to slide back and forth in the direction of the longitudinal axis in said interior space of the guiding member, wherein particularly the handle is connected to the guiding member. Particularly, the slider can be glued or otherwise connected (such as welded) to the bottom side of the outer surface of the tube. Such embodiments of the present invention can be used to move the tube relative to the fixed tool insert.

However, according to an alternative preferred embodiment of the present invention, the slider is connected to the tool insert, wherein the actuating mechanism is configured to move the slider back and forth in the direction of the longitudinal axis with respect to the tube to operate the tool insert, wherein particularly the handle is connected to the tube. Here, the slider can be glued or otherwise connected (such as welded) to the tool insert to move the tool insert relative to the fixed tube.

Furthermore, according to yet another preferred embodiment, the guiding member (for guiding the movable tube) encompasses the tube at least partially in a circumferential direction of the tube. Particularly, in a preferred embodiment, the guiding member is formed as a cylindrical sleeve having a wall delimiting the interior space of the guiding member, wherein optionally the wall can comprise a continuous gap extending along the longitudinal direction, the gap exposing a portion of the top side of the outer surface of the tube. Particularly, said portion of the top side of the outer surface of the tube comprises the maximum apex of said portion of the top side. Thus, the gap participates in improving sight along the top side of the tube of the surgical instrument.

One advantage of the present invention is, that the tube can be used with various tool inserts performing different functions.

Accordingly, in a preferred embodiment, the tool insert of the surgical instrument comprises a tool having two scissor blades configured to move, particularly pivot, towards one another to perform a cutting operation when the tube is moved relative to the scissor blades by the slider and pushes against the scissor blades. Thus, here, particularly, the tool insert forms a pair of scissors.

Further, in a preferred embodiment, the tool insert of the surgical instrument comprises a tool having a movable scissor blade configured to be moved by the slider relative a fixed scissor blade fixed to the tube to perform a cutting operation. Here, particularly, the tool insert forms part of a so-called vertical pair of scissors.

Furthermore, in a preferred embodiment, the tool insert of the surgical instrument comprises a movable blade configured to be moved by the slider relative to the tube from a retracted position in which the movable blade is arranged in the lumen of the tube to an advanced position in which the movable blade protrudes out of the lumen of the tube at the distal end of the tube, wherein particularly the distal end is atraumatic. Here, particularly, the distal end of the tube forms a dissector when the movable blade is in the retracted position.

According to yet another preferred embodiment of the present invention, the handle comprises a handle support configured to guide said back and forth movement of the slider.

Particularly, in a preferred embodiment, the handle support comprises a first support member and a second support member, wherein particularly the second support member forms a front cap of the handle being arranged on the first support member. Particularly, the first support member forms a central bar of the handle.

According to a further preferred embodiment of the present invention, the slider comprises a slider body and a first and a second wing hinged to the slider body, respectively. Further, in a preferred embodiment, the slider comprises a connector via which the slider body is connected to the tube or the tool insert. Particularly, the connector can be glued to the tube or tool insert or can be connected thereto in an alternative fashion (e.g. by a welding connection).

Particularly, in a preferred embodiment, the slider body comprises an (e.g. cylindrical) end section guided in the second support member (e.g. front cap), and wherein the wings are hinged to a middle section of the slider. Particularly, in a preferred embodiment the middle section of the slider body is arranged in an opening of the connector, particularly in a form-fitting manner, wherein the connector is guided in the second support member. In a preferred embodiment, the surgical instrument comprises a spring acting on the slider body for exerting a restoring force on the slider body. The spring may pull on the slider body or push the slider body to provide said restoring force.

According to a further preferred embodiment, the slider body and the connector may be integrally connected to one another (i.e. form a monolithic part that may be manufactured by way of injection molding) instead of said form-fitting connection.

In a preferred embodiment, the spring is arranged on the end section of the slider body, wherein particularly the second support member (e.g. front cap) of the handle support forms a stop for the spring. However, the spring may also be arranged on the other side of the first support member so that a portion of the first support member is arranged between the slider and the spring.

According to yet another preferred embodiment, the handle comprises a first and a second manually operable actuating member connected to the handle support, respectively. Particularly, the actuating members are connected, particularly in a pivotable or bendable fashion, to the first support member, particularly such that the first support member is arranged between the two actuating members which can be pivoted or bent towards the first support member to actuate the surgical instrument.

In a preferred embodiment, the first actuating member is configured to be pivoted or bent to push against the first wing of the slider, and wherein the second actuating member is configured to be pivoted or bent to push against the second wing of the slider to move the slider, particularly against the action of the spring which provides a restoring force and pushes the slider back into its initial position when the actuating members are released.

Furthermore, in a preferred embodiment of the present invention, the tool insert is rotatably supported on the handle support, particularly on the first support member, and wherein the surgical instrument comprises a lever connected to a proximal end section of the tool insert, the lever being manually operable to rotate the insert about the longitudinal axis, wherein particularly the lever does not extend in the vertical direction past the maximum apex of the top side of a proximal portion of the tube when the surgical instrument is in the at least one operating state.

According to a further preferred embodiment, the tool insert comprises a shaft accommodated at least partially in the lumen of the tube, wherein particularly the shaft extends along the longitudinal axis.

In a preferred embodiment, for further facilitating a view along the top side of the tube being unobstructed by a distal end of the tool insert, the shaft of the insert extends along the longitudinal axis and the tool is arranged at an angle to the shaft.

In a preferred further embodiment, the tube comprises a distal portion connected to a proximal portion, the distal portion being curved away from the longitudinal axis, and wherein a distal end section of the shaft of the tool insert is curved, wherein particularly the distal end section of the shaft comprises a reduced bending stiffness compared to an adjacent proximal section of the shaft, particularly for preventing the curved distal end section of the shaft from getting stuck in the lumen of the tube, particularly in the vicinity of the curved distal portion of the tube and its lumen.

Furthermore, according to a preferred embodiment of the present invention, the tube comprises a distal portion adjoining a proximal portion of the tube, wherein the distal portion is tapered with respect to the proximal portion, i.e., comprises an outer diameter being smaller than an outer diameter of the proximal portion. Particularly, the distal portion can be tapered in a continuous or a stepwise fashion.

According to yet another preferred embodiment, the handle support, particularly the first support member, comprises an elongated portion which particularly comprises said opposing end of the surgical instrument. Particularly, the actuating members are hinged to said elongated a portion of the handle support, wherein particularly said elongated portion extends along a further longitudinal axis that forms an angle with the longitudinal axis of the tube. Particularly, this angle between of the longitudinal axis of the tube and the further longitudinal axis of the elongated portion of the handle support is in the range between 15° and 90°, particularly 20° to 60°, particularly 20° to 40°, wherein particularly said angle is about 30°.

According to yet another preferred embodiment, the angle between the longitudinal axis of the tube and the further longitudinal axis of the elongated portion of the handle support is adjustable, particularly such that said angle can assume a value within one of the above stated ranges.

Particularly, in an embodiment, said elongated portion of the handle support can be pivotable so as to allow adjustment of said angle between the further longitudinal axis of the elongated portion of the handle support and the longitudinal axis of the tube.

Furthermore, particularly, re-usable instruments are still used almost exclusively in neurosurgery. According to a preferred embodiment, the surgical instrument is configured to be re-used, i.e., sterilized. However, according to a preferred alternative embodiment, the surgical instrument according to the present invention is a single-use surgical instrument, particularly for the following reasons. Blood and other body fluids may enter the tube via the tip of the surgical instrument during an operation. It is very difficult to clean the surgical instruments (without disassembly) without leaving any residue. However, in case of a single-use (i.e. disposable) surgical instrument, cross-contamination or infection due to residues can be ruled out.

Furthermore, scissors or other blades of re-usable tool inserts for example (see e.g. above), lose their sharpness with each procedure. They must therefore be sharpened at regular intervals. With a single-use surgical instrument, the necessary cutting quality is always guaranteed. Furthermore, the quality of a steel used for such scissors/blades can be lower as only a limited number of cuts are made with them. Thus, no reprocessing is necessary. This saves on equipment, its maintenance, material and personnel.

Furthermore, a further aspect of the present invention relates to a surgical instrument, comprising:
- a tube extending along a longitudinal axis from a proximal end to a distal end of the tube, wherein the tube comprises a circumferential wall delimiting a lumen of the tube, wherein the circumferential wall comprises an outer surface facing away from the lumen, the outer surface comprising a top side and a bottom side, and
- a handle for holding and manually operating the surgical instrument, the handle being operatively connected to the tube, the handle extending along a vertical axis of the surgical instrument on the bottom side of the tube, the vertical axis extending orthogonal to the longitudinal axis and pointing away from the top side of the tube, wherein the bottom side is arranged below the top side in the direction of the vertical axis.

Particularly, all features and embodiments of the first aspect of the present invention described herein can be used to further characterize the further aspect according to the present invention. Particularly, the subject-matter of each of the claims 3 to 15 or combinations thereof can be used to further characterize the further aspect according to the present invention.

In the following, embodiments of the present invention as well as further features and advantages of the present invention shall be described with reference to the Figures, wherein
- Fig. 1: shows an access system C as known in the prior art for providing a surgical access to an area of intervention E of a brain of a person along an axis A, wherein the surgical instrument is typically entered along axis A so that its longitudinal axis more or less aligns with the depicted axis A;
- Fig. 2: shows a perspective view of an embodiment of a surgical instrument according to the present invention;
- Fig. 3: shows a perspective view of certain parts of the surgical instrument shown in Fig. 2, particularly an elongated tube for actuating a tool insert arranged therein, a connector for moving the tube with respect to the tool insert, and a lever for rotating the tool insert about the longitudinal axis of the tube with respect to the tube;
- Fig. 4: shows a top view in the direction of the longitudinal axis of the tube onto the tube and the connector shown in Fig. 3 indicating the apex of the top side of the tube and the minimal superstructure protruding beyond the apex in the vertical direction;
- Fig. 5: shows a perspective view of the parts shown in Fig. 3, wherein in addition to Fig. 3 a slider body accommodated in an opening of the connector is shown as well as a coil spring arranged on the slider body, wherein the connector and the slider body form a slider that can be used by an actuating mechanism to move the tube, wherein the spring can provide a restoring force for returning the slider to an initial position;
- Fig. 6: shows a perspective view of the parts shown in Fig. 5, further depicting a handle support for a handle of the device, the handle support allowing to guide a movement of the slider along the longitudinal axis of the tube;
- Fig. 7: shows a perspective of the whole assembly shown in Fig. 6;
- Fig. 8: shows a perspective view of the assembly of Fig. 6 further illustrating that the handle support comprises a first support member configured to hold actuating members for actuating the slider (not shown in Fig. 8) and for rotatably supporting the tool insert, and a second support member connecting the handle support to a guiding member guiding the movement of the tube, wherein the second support member forms a front cap of the handle of the instrument and is connected to the first support member and guides the slider together with the first support member;
- Fig. 9: shows a cross-sectional view along the longitudinal axis of the tube of the assembly shown in Fig. 8;
- Fig. 10: shows a perspective top view onto the embodiment shown in Fig. 1 also indicating two actuating members connected to the handle support for actuating the slider shown in Figs. 5 to 9;
- Fig. 11: shows a detail of the embodiment of Fig. 10;
- Fig. 12: shows a detail of Fig. 11 with the first support member and the second support member (front cap) removed to show the interaction of the actuating members of the handle of the surgical instrument with wings of the slider allowing to move the slider along the longitudinal axis of the tube against the action of the spring;
- Fig. 13: shows a plan view onto the embodiment of the surgical instrument according to the present invention shown inter alia in Fig. 1 in the direction of the longitudinal axis of the tube as typically seen by a surgeon upon using the surgical instrument;
- Fig. 14: shows a schematic illustration of an embodiment of a tool of a tool insert of the surgical instrument, wherein the tool comprises two scissor blades configured to be pivoted towards and away from another to perform a cutting operation by movement of the tube with respect to the tool insert;
- Fig. 15: shows a schematic illustration of a further embodiment of a tool of a tool insert of the surgical instrument, wherein the tool comprises a movable scissor blade and a fixed scissor blade being fixed to the tube;
- Fig. 16: shows a further embodiment of a tool insert of the surgical instrument according to the present invention, wherein the tool, particularly comprising two scissor blades, is arranged at an angle with respect to the longitudinal axis of the tube;
- Fig. 17: shows a further embodiment of a surgical instrument according to the present invention, wherein the tube comprises a tapered distal end portion;
- Fig. 18: shows a further embodiment of a surgical instrument according to the present invention, wherein the tube and the tool insert comprise a curved distal end portion, respectively;
- Fig. 19: shows a further embodiment of a surgical instrument according to the present invention, wherein the tube comprises a curved distal end portion, wherein a shaft of the tool insert comprises a distal end section having reduced stiffness; and
- Fig. 20: shows a variant of the embodiment shown in Fig. 19.

The present invention relates to a surgical instrument 1, particularly a neurosurgical instrument, that can be used with a known access system C as shown in Fig. 1.

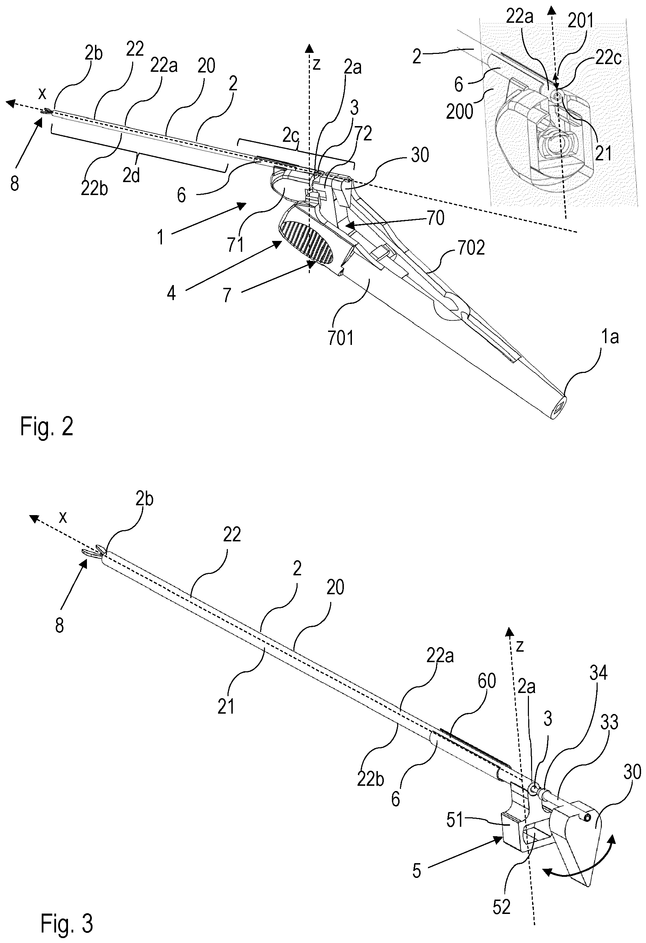

According to an embodiment of the surgical instrument 1 as shown in Fig. 2, the surgical instrument 1 comprises a tube 2 extending along a longitudinal axis x from a proximal end 2a of the tube 2 to a distal end 2b of the tube 2. In the framework of the present invention, the notion distal and proximal relate to positions with respect to the handle 7, wherein proximal components are closer to the handle 7 (or to a user operating the handle), while distal components are farther away from the handle 7 compared to a proximal counterpart.

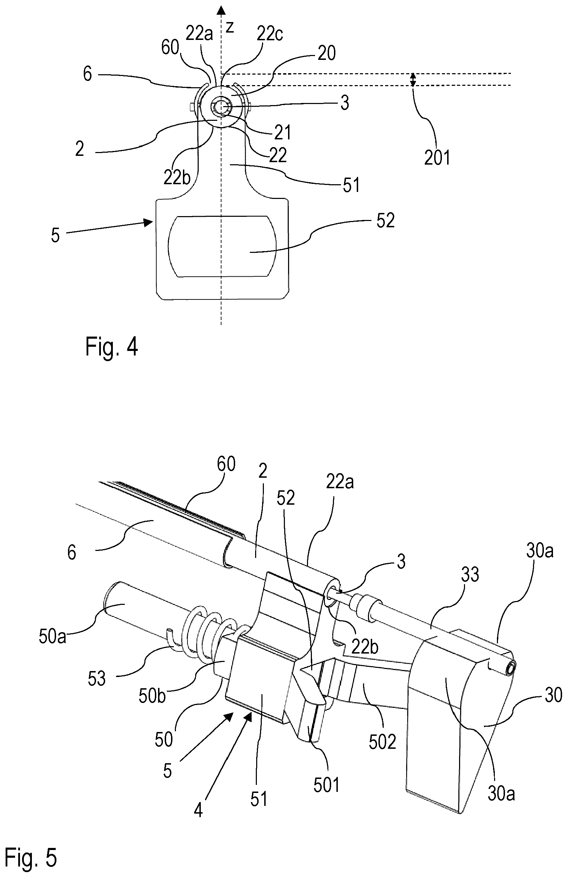

The tube 2 preferably comprises an elongated cylindrical shape and comprises a circumferential wall 20 defining an (e.g. cylindrical) lumen 21 of the tube 2 that extends from the proximal end 2a to the distal end 2b of the tube 2. Furthermore, the circumferential wall 20 comprises an outer surface 22 facing away from the lumen 21, the outer surface 22 comprising a top side 22a and a bottom side 22b (cf. also Fig. 4). Further, the surgical instrument 1 comprises a handle 7 configured for holding and manually operating the surgical instrument 1. To this end, the handle 7 is operatively connected to the tube 2 and extends along a vertical axis z of the surgical instrument 1 on the bottom side 22b of the tube 2 as e.g. shown in Fig. 2. Particularly, in this regard, it is not mandatory that the handle extends purely parallel to the vertical axis z and can also have additional directions of extensions as shown in e.g. in Fig. 2.

The vertical axis z runs perpendicular to the longitudinal axis x and points away from the top side 22a of the tube 2 as indicated in Figs. 2 and 4 for example. Accordingly, the bottom side 22b is arranged below the top side 22a in the direction of the vertical axis z.

Particularly, the tube 2 is configured to accommodate a tool insert 3 in its lumen 21 that can comprise a tool 8 at a distal end of the tool insert (cf. Figs. 2 - 3). In certain preferred embodiments, the tool 8 can be operable by moving the tube 2 along the longitudinal axis x with respect to the tool insert 3, wherein such a movement of the tube 2 can be actuated by an actuating mechanism that can be activated by a user via the handle 7. In alternative preferred embodiments the tool 8 can be operable by moving the tool insert 3 along the longitudinal axis x with respect to the tube 2. Both ways of operating the tool 8 will be described further down below in more detail. Alternative tools can also be used and operated with the handle 7 (see also below).

As the space in an access system C such as shown in Fig. 1 is typically very narrow in a plane extending perpendicular to the axis A, it is desirable that the surgical instrument 1 - once introduced into the access system C along axis A such that the longitudinal axis x of the tube 2 essentially extends along axis A - allows an unobstructed view along the top side 22a of the tube 2 in the direction of the longitudinal axis x. To this end, the surgical instrument 1 comprises at least one operating state in which the surgical instrument 1 extends no more than a distance 201 of 4 mm beyond a maximum apex 22c of the top side 22a of the tube 2 in the direction of the vertical axis z in any cross-section of the surgical instrument 1 that extends perpendicular to the longitudinal axis x and is arranged between the distal end 2b of the tube 2 and an opposing end 1a of the surgical instrument 1. Particularly, the inset of Fig. 2 in the upper right portion of Fig. 2 shows an example of one of the afore-mentioned cross sections showing the top side 22a of the tube with its maximum apex 22c. Also indicated is the distance 201. Furthermore, Fig. 4 which shows the tube 2 and a connector 5 connected thereto which serves for moving the tube 2 by means of the handle 7 also indicates the maximum apex 22c of the top side 22a of the tube 2 and said distance 201. As any superstructure comprises a height less than said distance 201, the design of the surgical instrument 1 permits a sufficient view along the top side 22a of the tube 2 of the vicinity of the distal end 2b of the tube 2 where the tool 8 is situated. This allows the surgeon an optimal handling of the instrument 1 as the field of vision is not blocked by any superstructure protruding beyond said preferred distance 201. Optimally, as has been found in experiments, the distance 201 is smaller or equal to 4 mm, preferably smaller or equal to 3 mm, preferably smaller or equal to 2 mm, particularly smaller or equal to 1mm, preferably smaller or equal to 0.5 mm. However, due to the design of the surgical instrument 1, said distance 201 can also be made to vanish, i.e., the surgical instrument 1 may also not extend beyond the maximum apex 22c of the top side 22a of the tube 2 at all in the direction of the vertical axis z in any cross-section of the surgical instrument 1 that extends perpendicular to the longitudinal axis x and is arranged between the distal end 2b of the tube 2 and the opposing end 1a of the surgical instrument 1 in certain preferred embodiments.

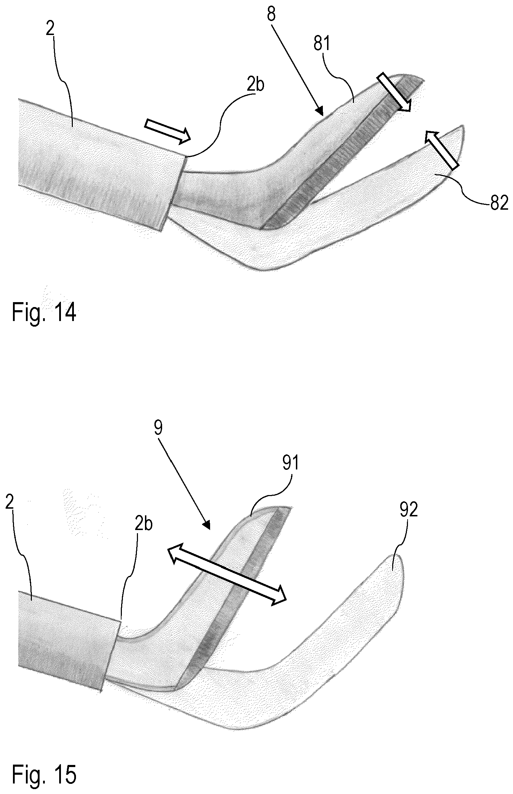

Furthermore, in certain preferred embodiments, the surgical instrument 1 has a multifunctional design in that the surgical instrument 1 can be used with different tool inserts 3. Particularly, the tool insert 3 can comprise a tool 8 such as scissors (cf. e.g. Figs. 14, 19-20), particularly so-called vertical scissors 9 (cf. e.g. Fig. 15), a retractable blade (so that the surgical instrument 1 can be used a scalpel and dissector), or a similar tool that is operated, e.g., closed and reopened, by the relative movement with the tube 2. Particularly, the handle 7 and the tube 2 with the tool insert 3 are located at different spatial levels in order to increase the visibility of the distal end 2b of the tube 2 at which the respective tool 8, 9 (e.g. scissors etc.) is operated. Particularly, the longitudinal axis x forms a main axis of the surgical instrument 1 in which the tube 2 and the tool insert 3 extend and are particularly moved axially relative to the other, as will be described in more detail further down below.

Particularly, this can be achieved by an independently guided actuating mechanism that can be one of: mechanical, electrical, pneumatic, hydraulic etc. Particularly, the actuating mechanism is arranged parallel to the main axis / longitudinal axis x, and actuates and guides one or more components of the main axis x (e.g. the tube 2 or the tool insert 3) by coupling. Particularly, this arrangement of the actuating mechanism can be utilized to avoid excessive superstructures protruding from the top side 22a of the tube 2, thus allowing to keep the field of vision completely clear.

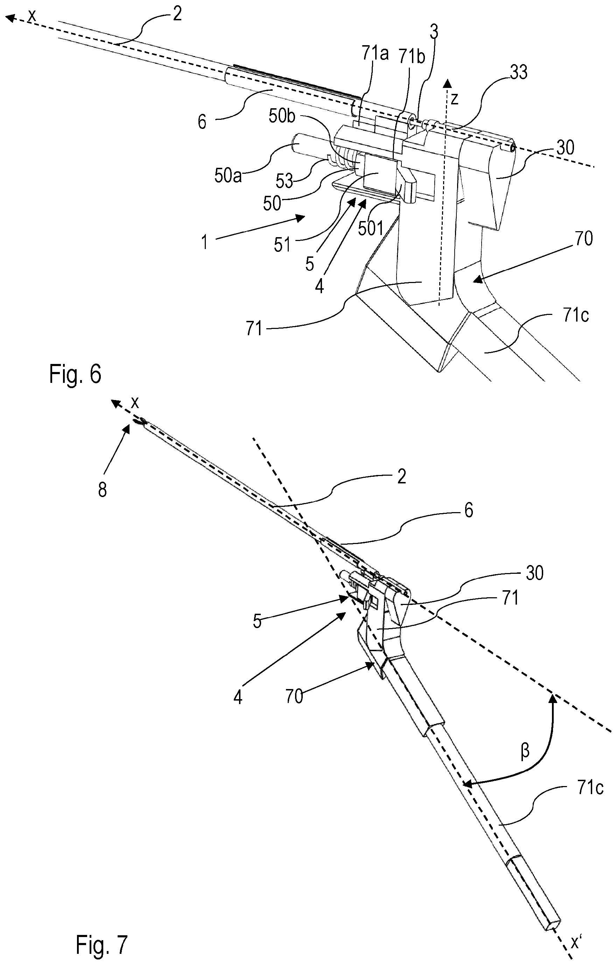

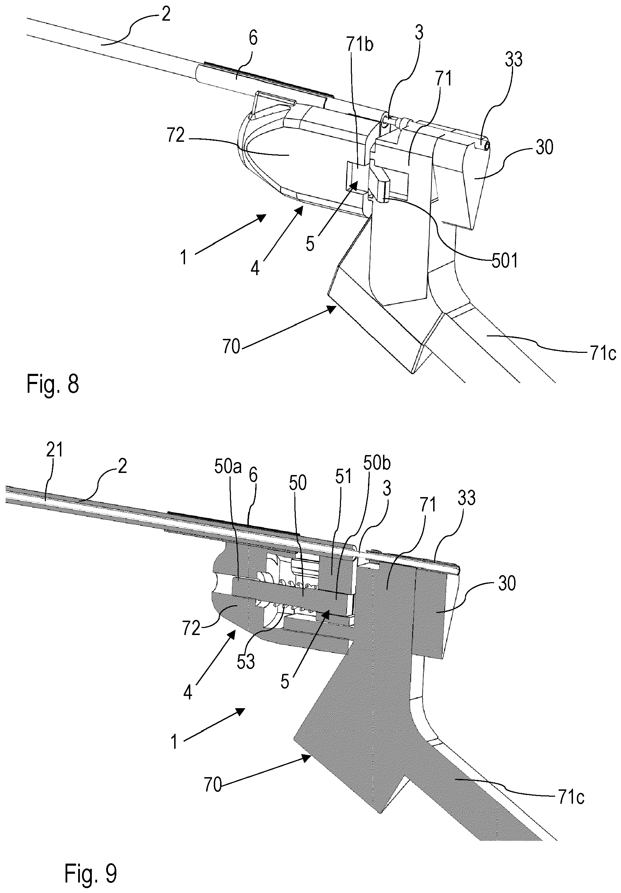

Fig. 3 shows a perspective view of certain parts of the surgical instrument shown in Fig. 2, namely the elongated tube 2, a tool insert 3 comprising a tool 8 such as a pair of scissors, a connector 51 of a slider 5 for moving the tube 2 with respect to the axially fixed tool insert 3. For guiding the tube 2 in the longitudinal axis x, a guiding member 6 is provided that is fixed to the handle support 70 (cf. e.g. Figs. 2 and 8). Furthermore, the tool insert 3 is firmly connected to a rotation tube 33 which in turn is connected to a handle support 70 shown e.g. in Figs. 5 and 6. Particularly, the rotation tube 33 can be rotated about the longitudinal axis x, but cannot move axially (i.e. along the longitudinal axis x) with respect to the handle support 70. Particularly, the rotation tube 33 comprises a ring member 34 that serves as a stop for preventing said axial movement of the rotation tube 33. Furthermore, the rotation tube 33 and therewith the tool insert 3 can be rotated about the longitudinal axis x by means of a lever 30 that is connected to the rotation tube 33.

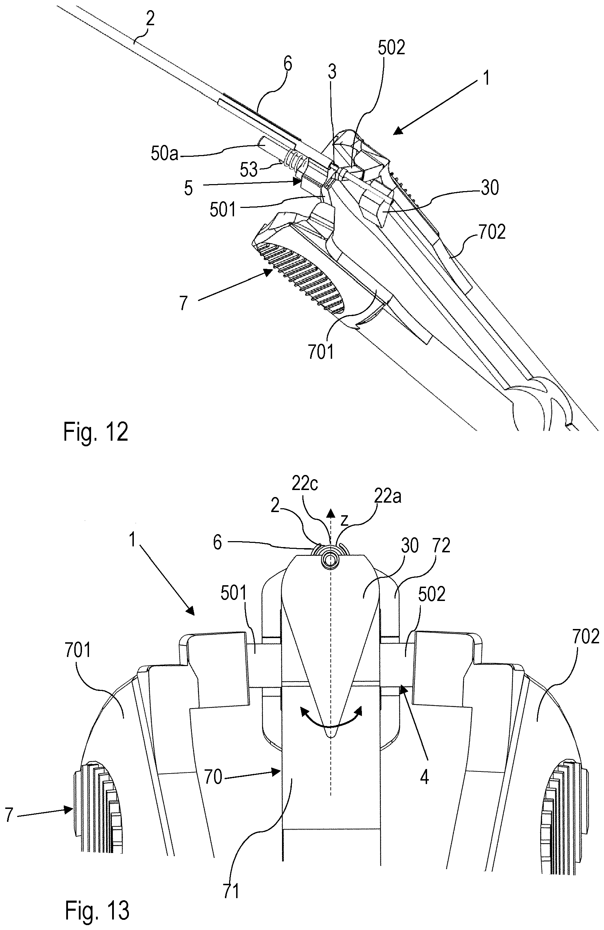

Depending on a rotation angle of the lever 30 (cf. e.g. Fig. 13), the surgical instrument 1 may comprise an operation state in which the lever 30 protrudes above the apex 22c of the top side 22a in the vertical axis z by more than the preferred distance 201 indicated in Figs. 2 and 4. However, for moderate rotation angles, operating states exist in which the lever 30 does not protrude by more than said distance 201. Furthermore, the rotation mechanism is an optional feature of the instrument 1 and may be completely omitted. Furthermore, the shape of the handle 30 can be adapted to allow for an even wider range of rotation angles that do not interfere with the sight along the top side 22a of the tube. Particularly, the curved upper edges 30a of the lever 30 are provided to this end and allow for a sufficient rotation angle of the lever 30 / tool insert 3 that does not interfere with the sight along the top side 22a of the tube 2.

Fig. 4 shows a further view of a portion of the assembly according to Fig. 3 in the direction of the longitudinal axis x also indicating the maximum apex 22c of the top side 22a of the tube 2 and the minimal superstructure, here given by portions of the guiding member 6 protruding beyond the maximum apex 22c in the vertical direction z. As can be inferred from Fig. 4, the connector 51 of the slider 5 comprises an upper portion via which the connector 51 is connected (e.g. glued or welded) to the bottom side 22b of tube 2. The upper portion of the connector 51 that connects to the tube 2 particularly comprises a width corresponding to an outer diameter of the tube 2. Furthermore, the upper portion of the connector 51 is integrally connected to a lower portion of the connector 51 which comprises an opening 52, particularly in form of a through-opening 52 that particularly extends through the connector 51 in the direction of the longitudinal axis x. Particularly, the lower portion of the connector 51 comprises a width perpendicular to the longitudinal axis x and the vertical axis z that is larger than a width of the upper portion in the same direction.

Furthermore, particularly, the guiding member 6 encompasses the tube 2 at least partially in a circumferential direction of the tube 2, wherein, as indicated, the guiding member 6 can be formed as a cylindrical sleeve having a wall delimiting the interior space of the guiding member 6, wherein optionally the wall can comprise a continuous gap 60 extending along the longitudinal direction x. The gap 60 exposes a portion of the top side 22a of the outer surface of the tube 2 and thus allows viewing along the apex 22c of the top side 22a as the sleeve 6 does not cover the maximum apex 22c in the vicinity of the gap 60.

Furthermore, Fig. 5 shows in addition to the components described in conjunction with Fig. 3 and 4 a slider body 50 accommodated in an opening 52 of the connector 51 (e.g. in form-fitting manner) as well as a spring 53 (e.g. coil spring) arranged on an (e.g. cylindrical) end section 50a of the slider body 50, wherein the connector 51 and the slider body 50 together form a slider 5 that can be used as part of an actuating mechanism 4 to move the tube 2 in the longitudinal direction x, wherein the spring 53 can provide a restoring force for returning the slider 5 to an initial position. Particularly, the end section 50a extends parallel to the longitudinal axis x. Particularly, according to a preferred embodiment, the slider body 50 and the connector 51 may also be integrally connected to one another, i.e., may form a monolithic part, that may be made by injection molding.

Particularly, the actuating mechanism 4 can be configured to move the slider 5 back and forth in the direction of the longitudinal axis x with respect to the tool insert 3 to operate the tool insert 3, wherein the tube 2 is guided by said guiding member 6 and wherein particularly the handle 7 of the surgical instrument 1 is firmly connected to the guiding member 6.

Alternatively (not shown in Fig. 5) the slider 5 can instead be connected to the tool insert 3, to move the tool insert 3 back and forth in the direction of the longitudinal axis x with respect to the tube 2 to operate the tool insert 3, wherein particularly the handle 7 is connected to the tube 2.

As further shown in Fig. 5, the slider 5 comprises a first and a second wing 501, 502 hinged to a middle section 50b of the slider body 50, respectively. The wings 501, 502 can be integrally connected to said middle section 50b via a living hinge, respectively.

Now, regarding actuation and guiding of the slider 5, Figs. 6 to 9 particularly illustrate guiding of the slider 5 by a handle support 70 of the handle 7 of the surgical instrument 1, which handle support 70 further supports a first and a second actuating member 701, 702 (as shown e.g. in Figs. 1, 10 to 13) forming part of the actuating mechanism 4. The actuating members 701, 702 are configured to be manually pressed, particularly pivoted, or bent, towards one another to interact with the wings 501, 502 of the slider 5 causing the slider 5 to move forward in the direction of the longitudinal axis x thereby taking along the tube 2.

Particularly, as indicated in Fig. 6 and 7 the handle support 70 comprises a first support member 71, which particularly provides a recess for accommodating the slider 5 in a sliding fashion to guide the latter along the longitudinal axis x. Particularly, the first support member 71 comprises a top aperture 71a through which the upper portion of the connector 51 protrudes to connect to the bottom side 22b of the tube 2 and in which the upper portion of the connector 51 can slide in a guided fashion along the longitudinal axis x. Furthermore, the first support member 71 comprises two opposing lateral apertures 71b allowing the wings 501, 502 to protrude out of the first support member 71 in direction running orthogonal to both the longitudinal axis x and the vertical axis z.

Furthermore, the first support member 71 also comprises an elongated portion 71c extending along a further longitudinal axis x' that forms an angle β with the longitudinal axis x of the tube 2. Particularly, this angle β between the longitudinal axis x of the tube 2 and the further longitudinal axis x' of the elongated portion 71c of the first support member 71 is in the range between 15° and 90°, particularly 20° to 60°, particularly 20° to 40°, wherein particularly said angle is about 30°. Furthermore, in certain embodiments said elongated portion 71c of the handle support 70 can be pivotable to allow adjustment of said angle β.

As shown in Figs. 8 and 9, the handle support 70 of the handle 7 of the surgical instrument 1 can further comprise a second support member 72 that is connected to the first support member 71 and preferably covers the slider 5 (e.g. in form of a front cap) while leaving the lateral apertures 71b open in a manner that the wings 501, 502 are still accessible from the outside (cf. Fig. 8).

Furthermore, as indicated in Fig. 9, the second support member 72 (e.g. front cap) is configured to also guide the slider 5, particularly said end section 50a, wherein the second support member 72 can also form a stop for the spring 53. Thus, when the slider 5 gets pressed forward (e.g. by the actuating members 701, 702), the coil 53 will butt against this stop and thus provide a restoring force acting on the slider 5.

A further function of the handle support 70, particularly of the first support member 71 is to support the first and the second actuating member 701, 702.

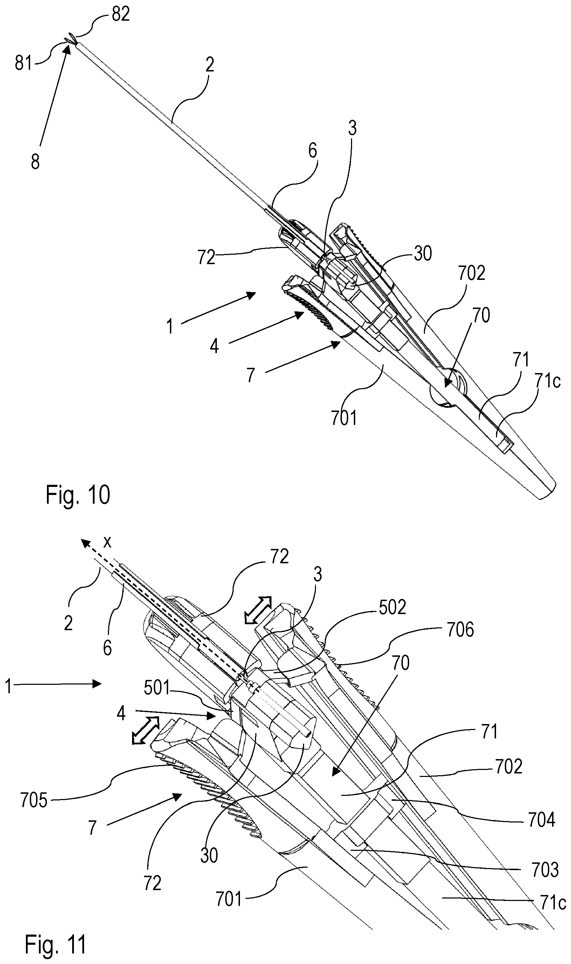

Particularly, the actuating members 701, 702 can be connected at a first end to form an opening into which an end of the elongated portion 71c of the first support member 71 can be inserted to connect the actuating members 701, 702 to the handle support 70. The actuating members 701, 702 can be fixed to the elongated portion 71c of the first support member 71 in a suitable fashion, e.g. by an adhesive, a press fit, a weld seam or another suitable connection. As indicated in Figs. 10 and 11, the opposing second ends of the actuating members 701, 702 can comprise a dented and/or surface structured region 705, 706, allowing to press the actuating members 701, 702 towards one another, particularly along an axis that is orthogonal to the longitudinal axis x as well orthogonal to the vertical axis z. The actuating members 701, 702 are each coupled to an associated wing 501, 502 of the slider 5 (cf. Fig. 11) so that pressing the actuating members 701, 702 towards one another presses the wings 501, 502 towards one another which causes the slider 5 and therewith the whole tube 2 to move distally in the direction of the longitudinal axis x. This will cause the coil 53 to press against the stop provided by the second support member 72 (cf.

Fig. 9) so that the coil 53 will be tensioned and the slider 5 will be returned to its initial position once the actuating members 701, 702 are released. The forward movement of the tube 2 in the direction of the longitudinal axis x can be used to operate a tool 8 arranged at a distal end of the tool insert 3 as e.g. shown in Fig. 10. Particularly the tool 8 can comprise two pivotable scissor blades 81, 82 that are pivoted towards one another to perform a cutting movement when the tube presses against them upon its forward movement. Moving the tube 2 back causes the scissor blades 81, 82 to open again. Alternative tools that can be used with the present invention will be described further down below.

Fig. 14 shows a schematic illustration of an embodiment of a tool 8 of a tool insert 3 of the surgical instrument 1, wherein the tool 8 comprises two scissor blades 81, 82 as described above that are configured to be pivoted towards and away from another to perform a cutting operation by movement of the tube 2 with respect to the tool insert 3. This movement of the tube 2 can be initiated as describe above, causing the distal end 2b to push against the blades 81, 82 which then close accordingly to perform a cutting movement.

Fig. 15 shows a schematic illustration of a further alternative embodiment of a tool 9 of a tool insert 3 of the surgical instrument 1, wherein here the tool 9 comprises a movable scissor blade 91 and a fixed scissor blade 92 being fixed to the tube 2. Here, the tube 2 is not moved as described above, but the tool insert 3 which comprises the movable blade 91 at its distal end. The tool insert 3 is connected to the slider 5 which can be actuated by the actuating members 701, 702 as described above.

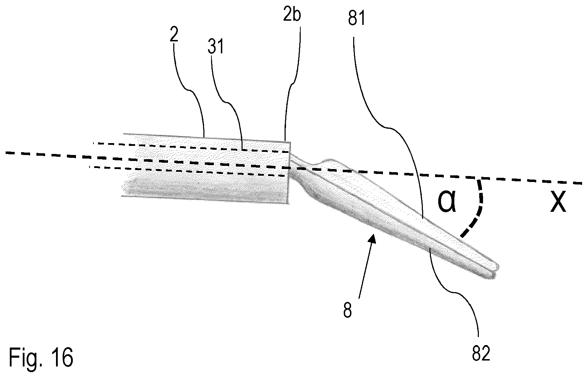

Fig. 16 shows a further embodiment of a tool insert 3 of the surgical instrument 1 according to the present invention, wherein the tool 8, particularly comprising two scissor blades 81, 82 as described above. Particularly, the tool insert 3 comprises a shaft 31 accommodated at least partially in the lumen 21 of the tube 2, wherein particularly the shaft 31 extends along the longitudinal axis x. For further facilitating a view along the top side 22a of the tube 2 being unobstructed by a distal end of the tool insert 3 the shaft 31 of the tool insert 3 extends along the longitudinal axis x and the tool 8 is arranged at an angle α to the shaft 31.

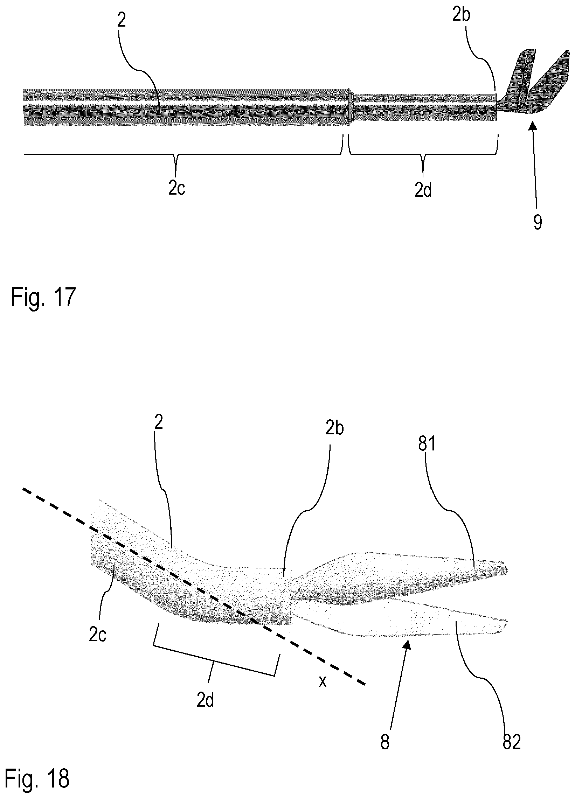

Fig. 17 shows a further embodiment of a surgical instrument 1 according to the present invention, wherein the tube 2 comprises a distal portion 2d adjoining a proximal portion 2c of the tube 2, wherein the distal portion 2d is tapered with respect to the proximal portion 2c, i.e., comprises an outer diameter being smaller than an outer diameter of the proximal portion 2c. Particularly, the distal portion 2d can be tapered in a continuous or a stepwise fashion.

Fig. 18 shows a further embodiment of a surgical instrument 1 according to the present invention, wherein the tube 2 comprises a curved distal end portion 2d that adjoins to a proximal straight portion 2c that extends in the direction of the longitudinal axis x. This configuration can also be used to improve sight along the top side 22a.

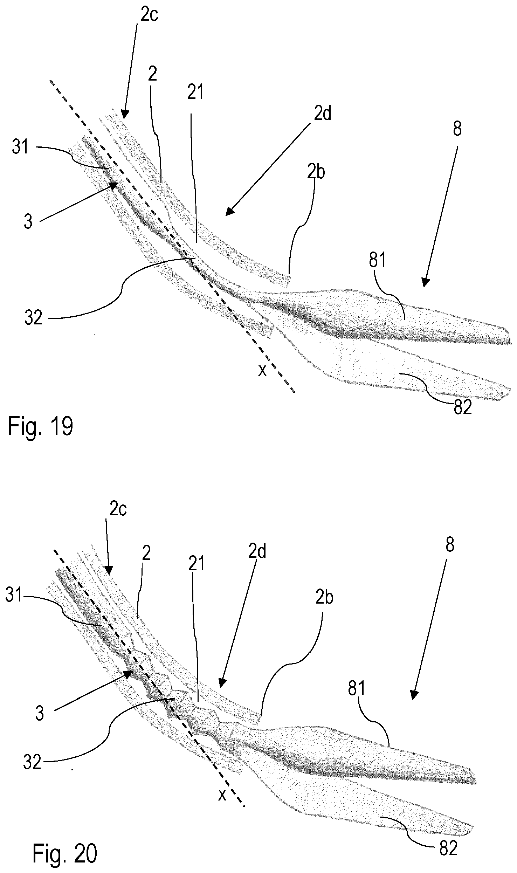

Particularly, as shown in Fig. 19 the tube 2 comprises a curved distal end portion 2d, wherein a shaft 31 of the tool insert 3 comprises a distal end section 32 having reduced stiffness to facilitate sliding of the tool insert 3 in the lumen 21 of the tube 2 in the vicinity of the curved distal portion 2d. Particularly, the stiffness of the distal end section 32 can be reduced by decreasing an outer diameter of the distal end section 32 compared to an adjoining proximal section of the shaft 31.

Fig. 20 shows a variant of the embodiment shown in Fig. 19, where the reduced bending stiffness of the distal end section 32 is achieved by a specific shape of the distal end section 32 which may comprise a plurality of recesses such as constrictions shown in Fig. 20.

As alternative to the embodiments shown in Figs. 19 and 20, to reduce the bending stiffness, it is also an option to have a curved distal end section 32 so that the distal end section follows the course of the tube 2. Particularly, as the tube 2 is typically only pushed 1 mm to 2 mm in the axial direction to activate the tool insert 3, this is normally sufficient.

According to yet another preferred embodiment, the tube 2 can be used as a tool itself (even without a tool insert). Specifically, in certain embodiments, the tube 2 can be configured to guide a fluid medium. In this case, the surgical instrument 1 is particularly configured to draw off a liquid medium and/or discharge a liquid medium through an opening of the tube 2 arranged at a distal end 2b of the tube 2, wherein said opening can be located on the face side of the tube 2 at the distal end 2b or can be a lateral opening formed in said wall 20 of the tube 2.

Furthermore, according to a preferred embodiment, the surgical instrument 1, particularly when the tube 2 can draw off liquid as described above, the surgical instrument may comprise a movable blade configured to be moved back and forth in the lumen 21 of the tube 2 to shred material carried by the fluid medium sucked into the lumen 21 through said opening of the tube 2.

The surgical instrument is particularly suitable for the field of neurosurgery, but can also be used in otosurgery or ophthalmology. However, it can also be used to advantage in other delicate procedures where accessibility and optimal visibility play a decisive role (preferably for procedures under the microscope).

## Claims

1. A surgical instrument (1), comprising:
- a tube (2) extending along a longitudinal axis (x) from a proximal end (2a) to a distal end (2b) of the tube (2), wherein the tube (2) comprises a circumferential wall (20) delimiting a lumen (21) of the tube (2), wherein the circumferential wall (20) comprises an outer surface (22) facing away from the lumen (21), the outer surface (22) comprising a top side (22a) and a bottom side (22b),
- a handle (7) for holding and manually operating the surgical instrument (1), the handle (7) being operatively connected to the tube (2), the handle (7) extending along a vertical axis (z) of the surgical instrument (1) on the bottom side (22b) of the tube (2), the vertical axis (z) extending orthogonal to the longitudinal axis (x) and pointing away from the top side (22a) of the tube (2), wherein the bottom side (22b) is arranged below the top side (22a) in the direction of the vertical axis (z),
wherein for facilitating an unobstructed view along the top side (22a) of the tube (2) in the direction of the longitudinal axis (x), the surgical instrument (1) comprises at least one operating state in which the surgical instrument (1) extends no more than 4 mm beyond a maximum apex (22c) of the top side (22a) of the tube (2) in the direction of the vertical axis (z) in any cross-section of the surgical instrument (1) that extends perpendicular to the longitudinal axis (x) and is arranged between the distal end (2b) of the tube (2) and an opposing end (1a) of the surgical instrument (1).

2. The surgical instrument according to claim 1, wherein for facilitating the unobstructed view along the top side (22a) of the tube (2) in the direction of the longitudinal axis (x), the surgical instrument (1) does not extend beyond the maximum apex (22c) of the top side (22a) of the tube (2) in the direction of the vertical axis (z) in any cross-section of the surgical instrument (1) that extends perpendicular to the longitudinal axis (x) and is arranged between the distal end (2b) of the tube (2) and the opposing end (1a) of the surgical instrument (1).

3. The surgical instrument according to claim 1 or 2, wherein the tube (2) is configured to guide a fluid medium and/or tissue.

4. The surgical instrument according to one of the preceding claims, wherein the surgical instrument (1) comprises a tool insert (3) arranged at least partially in the lumen (21) of the tube (2) so that the tube (2) and the tool insert (3) can move relative to one another in the direction of the longitudinal axis (x) to operate the tool insert (3).

5. The surgical instrument according to one of the preceding claims, wherein the surgical instrument (1) comprises an actuating mechanism (4) configured to move the tube (2) and tool insert (3) relative to one another to operate the tool insert (3).

6. The surgical instrument according to one of the preceding claims, wherein the surgical instrument (1) comprises a slider (5) extending along the vertical axis (z) of the tool, wherein
- the slider (5) is connected to the bottom side (22b) of the tube (2), wherein the actuating mechanism (4) is configured to move the slider (5) back and forth in the direction of the longitudinal axis (x) with respect to the tool insert (3) to operate the tool insert (3), wherein particularly the surgical instrument (1) comprises a guiding member (6) having an interior space, wherein particularly the tube (2) is configured to slide back and forth in the direction of the longitudinal axis (x) in said interior space, wherein particularly the handle (7) is connected to the guiding member (6),
or
- wherein the slider is connected to the tool insert (3), wherein the actuating mechanism (4) is configured to move the slider (5) back and forth in the direction of the longitudinal axis (x) with respect to the tube (2) to operate the tool insert (3), wherein particularly the handle (7) is connected to the tube (2).

7. The surgical instrument according to one of the claims 4 to 6, wherein the tool insert (3) comprises one of:
- a tool (8) having two scissor blades (81, 82) configured to move, particularly pivot, towards one another to perform a cutting operation when the tube (2) is moved relative to the scissor blades (81, 82) and pushes against the scissor blades (81, 82),
- a tool (9) having a movable scissor blade (91) configured to be moved relative to a fixed scissor blade (92) fixed to the tube (2) to perform a cutting operation,
- a movable blade configured to be moved relative to the tube (2) from a retracted position in which the movable blade is arranged in the lumen (21) to an advanced position in which the movable blade protrudes out of the lumen (21) at the distal and of the tube (2), wherein particularly the distal end (2b) is atraumatic.

8. The surgical instrument according to claim 6 or according to claim 7 insofar referring to claim 6, wherein the handle (7) comprises a handle support (70) configured to guide the slider (5).

9. The surgical instrument according to claim 6 or according to one of the claims 7 to 8 insofar referring to claim 7, wherein the slider (5) comprises slider body (50) and a first and a second wing (501, 502) hinged to the slider body (50), respectively, and wherein particularly the slider (5) comprises a connector (51) via which the slider body (50) is connected to the tube (2) or the tool insert (3).

10. The surgical instrument according to one of the preceding claims, wherein the handle (7) comprises a first and a second manually operable actuating member (701, 702) connected to the handle support (70), respectively.

11. The surgical instrument according to claims 9 and 10, wherein the first actuating member (701) is configured to push against the first wing (501) of the slider (5), and wherein the second actuating member (702) is configured to push against the second wing (502) of the slider (5) to move the slider (5).

12. The surgical instrument according to one of the preceding claims, wherein the tool insert (3) is rotatably supported on the handle support (70), and wherein the surgical instrument (1) comprises a lever (30) connected to a proximal end section of the tool insert (3), the lever (30) being manually operable to rotate the tool insert (3) about the longitudinal axis (x), wherein particularly the lever (30) does not extend in the vertical direction past the maximum apex (22c) of the top side (22a) of the tube (2) in the vertical direction (z) when the surgical instrument (1) is in said at least one operating state.

13. The surgical instrument according to claim 4 or according to one of the claims 5 to 12 insofar referring to claim 4, wherein the tool insert (3) comprises a shaft (31) accommodated at least partially in the lumen (21) of the tube (2).

14. The surgical instrument according to claims 7 and 13, wherein the shaft (31) of the tool insert (3) extends along the longitudinal axis (x) and the tool (8) is arranged at an angle (α) to the shaft (31).

15. The surgical instrument according to claim 13, wherein, the tube (2) comprises a distal portion (2d) connected to a proximal portion (2c) of the tube (2), wherein the distal portion (2d) of the tube (2) is curved away from the longitudinal axis (x), and wherein a distal end section (32) of the shaft (31) of the tool insert (3) is curved, wherein particularly the distal end section (32) of the shaft (31) comprises a reduced bending stiffness compared to an adjacent proximal section of the shaft (31), particularly for preventing the curved distal end section (32) of the shaft (31) from getting stuck in the lumen (21) of the tube (2).

## Amended claims

### Amended claims in accordance with Rule 137(2) EPC.

1. A surgical instrument (1), comprising:
- a tube (2) extending along a longitudinal axis (x) from a proximal end (2a) to a distal end (2b) of the tube (2), wherein the tube (2) comprises a circumferential wall (20) delimiting a lumen (21) of the tube (2), wherein the circumferential wall (20) comprises an outer surface (22) facing away from the lumen (21), the outer surface (22) comprising a top side (22a) and a bottom side (22b),
- a handle (7) for holding and manually operating the surgical instrument (1), the handle (7) being operatively connected to the tube (2), the handle (7) extending along a vertical axis (z) of the surgical instrument (1) on the bottom side (22b) of the tube (2), the vertical axis (z) extending orthogonal to the longitudinal axis (x) and pointing away from the top side (22a) of the tube (2), wherein the bottom side (22b) is arranged below the top side (22a) in the direction of the vertical axis (z), wherein for facilitating an unobstructed view along the top side (22a) of the tube (2) in the direction of the longitudinal axis (x), the surgical instrument (1) comprises at least one operating state in which the surgical instrument (1) extends no more than 4 mm beyond a maximum apex (22c) of the top side (22a) of the tube (2) in the direction of the vertical axis (z) in any cross-section of the surgical instrument (1) that extends perpendicular to the longitudinal axis (x) and is arranged between the distal end (2b) of the tube (2) and an opposing end (1a) of the surgical instrument (1),
- a tool insert (3) arranged at least partially in the lumen (21) of the tube (2) so that the tube (2) and the tool insert (3) can move relative to one another in the direction of the longitudinal axis (x) to operate the tool insert (3), and
an actuating mechanism (4) configured to move the tube (2) back and forth in the direction of the longitudinal axis (x) with respect to the tool insert (3) to operate the tool insert (3).

2. The surgical instrument according to claim 1, wherein for facilitating the unobstructed view along the top side (22a) of the tube (2) in the direction of the longitudinal axis (x), the surgical instrument (1) does not extend beyond the maximum apex (22c) of the top side (22a) of the tube (2) in the direction of the vertical axis (z) in any cross-section of the surgical instrument (1) that extends perpendicular to the longitudinal axis (x) and is arranged between the distal end (2b) of the tube (2) and the opposing end (1a) of the surgical instrument (1).

3. The surgical instrument according to claim 1 or 2, wherein the tube (2) is configured to guide a fluid medium and/or tissue.

4. The surgical instrument according to one of the preceding claims, wherein the surgical instrument (1) comprises a slider (5) extending along the vertical axis (z) of the tool, wherein
the slider (5) is connected to the bottom side (22b) of the tube (2), wherein the actuating mechanism (4) is configured to move the slider (5) back and forth in the direction of the longitudinal axis (x) with respect to the tool insert (3) to operate the tool insert (3), wherein particularly the surgical instrument (1) comprises a guiding member (6) having an interior space, wherein particularly the tube (2) is configured to slide back and forth in the direction of the longitudinal axis (x) in said interior space, wherein particularly the handle (7) is connected to the guiding member (6),

5. The surgical instrument according to one of the preceding claims, wherein the tool insert (3) comprises one of:
- a tool (8) having two scissor blades (81, 82) configured to move, particularly pivot, towards one another to perform a cutting operation when the tube (2) is moved relative to the scissor blades (81, 82) and pushes against the scissor blades (81, 82),
- a tool (9) having a movable scissor blade (91) configured to be moved relative to a fixed scissor blade (92) fixed to the tube (2) to perform a cutting operation,
- a movable blade configured to be moved relative to the tube (2) from a retracted position in which the movable blade is arranged in the lumen (21) to an advanced position in which the movable blade protrudes out of the lumen (21) at the distal and of the tube (2), wherein particularly the distal end (2b) is atraumatic.

6. The surgical instrument according to claim 4 or according to claim 5 insofar referring to claim 4, wherein the handle (7) comprises a handle support (70) configured to guide the slider (5).

7. The surgical instrument according to claim 4 or according to one of the claims 5 to 6 insofar referring to claim 4, wherein the slider (5) comprises slider body (50) and a first and a second wing (501, 502) hinged to the slider body (50), respectively, and wherein particularly the slider (5) comprises a connector (51) via which the slider body (50) is connected to the tube (2) or the tool insert (3).

8. The surgical instrument according to one of the preceding claims, wherein the handle (7) comprises a first and a second manually operable actuating member (701, 702) connected to the handle support (70), respectively.

9. The surgical instrument according to claims 7 and 8, wherein the first actuating member (701) is configured to push against the first wing (501) of the slider (5), and wherein the second actuating member (702) is configured to push against the second wing (502) of the slider (5) to move the slider (5).

10. The surgical instrument according to one of the preceding claims, wherein the tool insert (3) is rotatably supported on the handle support (70), and wherein the surgical instrument (1) comprises a lever (30) connected to a proximal end section of the tool insert (3), the lever (30) being manually operable to rotate the tool insert (3) about the longitudinal axis (x), wherein particularly the lever (30) does not extend in the vertical direction past the maximum apex (22c) of the top side (22a) of the tube (2) in the vertical direction (z) when the surgical instrument (1) is in said at least one operating state.

11. The surgical instrument according to one of the preceding claims, wherein the tool insert (3) comprises a shaft (31) accommodated at least partially in the lumen (21) of the tube (2).

12. The surgical instrument according to claims 5 and 11, wherein the shaft (31) of the tool insert (3) extends along the longitudinal axis (x) and the tool (8) is arranged at an angle (α) to the shaft (31).

13. The surgical instrument according to claim 12, wherein, the tube (2) comprises a distal portion (2d) connected to a proximal portion (2c) of the tube (2), wherein the distal portion (2d) of the tube (2) is curved away from the longitudinal axis (x), and wherein a distal end section (32) of the shaft (31) of the tool insert (3) is curved, wherein particularly the distal end section (32) of the shaft (31) comprises a reduced bending stiffness compared to an adjacent proximal section of the shaft (31), particularly for preventing the curved distal end section (32) of the shaft (31) from getting stuck in the lumen (21) of the tube (2).
